# FASCICULE DE BREVET EUROPEEN

(11) **EP 3 946 052 B1**
(45) Date de publication et mention de la délivrance du brevet: **18.01.2023**
(21) Numéro de dépôt: 20713903.1
(22) Date de dépôt: 27.03.2020
(51) Int. Cl.: A61B 5/145, A61B 5/053, A61B 5/00, H02J 7/00

(54) **DISPOSITIF DE MESURE PORTABLE DE L'ÉTAT DE SANTÉ D'UN UTILISATEUR**
TRAGBARE VORRICHTUNG ZUR MESSUNG DES GESUNDHEITSZUSTANDS EINES BENUTZERS
PORTABLE DEVICE FOR MEASURING THE STATE OF HEALTH OF A USER

(30) Priorité: 29.03.2019 FR 1903314
(43) Date de publication de la demande: 09.02.2022
(73) Titulaire: ELLONA, 31400 Toulouse (FR)
(72) Inventeur: MIFSUD, Jean-Christophe, 82400 GOUDOURVILLE (FR)
(74) Mandataire: Argyma
(86) Numéro de dépôt international: PCT/EP2020/058861
(87) Numéro de publication internationale: WO 2020/201161

(56) Documents cités:
- WO-A2-2014/088768
- US-A1- 2012 101 430
- US-A1- 2016 038 055
- US-A1- 2016 192 856
- US-A1- 2017 023 518

## Description

### DOMAINE TECHNIQUE DE L'INVENTION

La présente invention se rapporte au domaine des dispositifs permettant la mesure de l'état de santé d'un utilisateur et qui sont portables.

De manière connue, un dispositif de mesure portable de l'état de santé est configuré pour être porté par un utilisateur et permet de mesurer différents indicateurs de santé de l'utilisateur pour contrôler l'état de santé dudit utilisateur. Pour cela, ce dispositif comprend au moins un organe de mesure ainsi qu'une batterie électrique d'alimentation de l'organe de mesure.

On connaît dans l'art antérieur une montre connectée comprenant un tel dispositif et configurée pour être portée au poignet de l'utilisateur. L'organe de mesure peut être un capteur optique permettant de mesurer le pouls ou le taux d'oxygène dans le sang, ou encore un capteur de température pour mesurer la température corporelle. La montre connectée comporte une batterie électrique qui peut être rechargée par un chargeur électrique. En pratique, la batterie comporte un connecteur de type prise USB.

Dans l'art antérieur, on connaît également par la demande de brevet FR2912049A1 une montre connectée comprenant un tel dispositif dont les organes de mesure sont deux électrodes de test configurées pour être au contact de la sueur du poignet de l'utilisateur. Ce dispositif comprend en outre un générateur envoyant un courant électrique entre les électrodes de test et un voltmètre mesurant l'impédance de la sudation de l'utilisateur. Cette mesure d'impédance permet de déterminer un éventuel état de stress ou de malaise de l'utilisateur. La batterie électrique est analogue à celle décrite précédemment.

On connaît par ailleurs par la demande de brevet US2016038055A1 un boîtier monté sur un bracelet comprenant deux contacts électriques au contact de la peau de l'utilisateur, un capteur de résistance, un capteur de capacité, un condensateur et une batterie électrique afin de mesurer la résistance, la capacité et le potentiel de Galvani de la peau de l'utilisateur. Les deux contacts électriques doivent comporter le même matériau pour pouvoir mesurer le potentiel de Galvani d'après la définition de l'IUPAC (International Union of Pure and Applied Chemistry). Un tel boîtier présente l'inconvénient de posséder un encombrement et un coût élevés et d'offrir des possibilités de mesure limitées.

De manière analogue, on connaît par la demande de brevet US2016/192856-A1 un boîtier comprenant deux électrodes pour mesurer une résistance de Galvani et une troisième électrode montée sur la face externe afin d'être atteignable par le doigt de l'utilisateur. Un capteur ECG permet de mesurer un électrocardiogramme entre la troisième électrode et l'une des deux électrodes de la face interne. Un tel boîtier présente les mêmes inconvénients que précédemment.

Par ailleurs, il est connu par la demande de brevet US2017023518A1 un boîtier comprenant plusieurs électrodes de référence couplées chacune à un transistor à effet de champ sélectif à un ion pour mesurer la concentration électrolytique de cet ion dans la sueur de l'utilisateur. Un tel boîtier présente un encombrement important car il nécessite des transistors à effet de champ pour rendre les électrodes de référence sélectives, qui ne le sont pas par nature. Chaque électrode permet en outre une unique mesure propre indépendante.

Un dispositif de mesure portable de l'état de santé permet ainsi d'informer son utilisateur sur son état de santé de manière immédiate. Il permet également de répondre à certaines des attentes des utilisateurs en matière de télémédecine face au manque de ressources médicales. Toutefois, un tel dispositif possède un encombrement important et un coût élevé, ce qui freine son adoption par le plus grand nombre. L'invention vise ainsi à résoudre au moins certains de ces inconvénients.

### PRESENTATION DE L'INVENTION

L'invention concerne un dispositif de mesure portable de l'état de santé d'un utilisateur configuré pour être porté par l'utilisateur, ledit dispositif comprenant au moins un organe de mesure et au moins une batterie électrique alimentant électriquement l'organe de mesure, ladite batterie électrique comprenant au moins deux électrodes de charge configurées pour coopérer avec un dispositif de charge afin de recharger électriquement la batterie électrique.

L'invention est remarquable en ce qu'au moins deux des électrodes de charge, dites électrodes de charge et de mesure, sont configurées pour être au contact de la sueur de l'utilisateur lorsque le dispositif est porté et sont reliées à l'organe de mesure afin de mesurer, en coopération avec ledit organe de mesure, au moins un paramètre physique de la sueur de l'utilisateur, afin de mesurer son état de santé.

De manière avantageuse, les électrodes de charge et de mesure remplissent, d'une part, une fonction de charge et, d'autre part, une fonction de mesure. Une telle double fonction permet avantageusement de réduire le nombre d'éléments par comparaison à l'art antérieur, ce qui limite le coût et l'encombrement. En outre, de manière avantageuse, lors d'une charge électrique via les électrodes de charge, celles-ci sont en contact physique avec le dispositif de charge, ce qui permet de nettoyer la surface des électrodes qui peuvent ainsi réaliser des mesures pertinentes.

De préférence, l'ensemble des électrodes de charge sont des électrodes de charge et de mesure. Autrement dit, il n'existe pas d'électrodes dédiées uniquement à la charge. Le nombre de mesures est avantageusement optimisé pour une masse et un encombrement réduits.

De manière préférée, les électrodes de charge et de mesure sont configurées pour mesurer la différence de potentiels électrolytiques en coopération avec l'organe de mesure. De manière avantageuse, la différence de potentiels électrolytiques est fonction du milieu dans lequel sont positionnées les électrodes de charge et de mesure, c'est-à-dire la sueur de l'utilisateur, ainsi que de la nature des électrodes de charge et de mesure.

De manière préférée, le dispositif comprend au moins un organe de calcul configuré pour déterminer au moins un indicateur de santé de l'utilisateur à partir de la différence de potentiels électrolytiques. La détermination de l'indicateur de santé est réalisée par calcul algébrique et/ou par recherche dans une base de données.

De manière préférée, le dispositif comprend au moins un organe de comparaison configuré pour comparer l'indicateur de santé à au moins un seuil de référence. Le seuil de référence est connu de l'organe de comparaison via la théorie, via des corrélations, ou via au moins un indicateur de santé déterminé précédemment. La comparaison de l'indicateur de santé au seuil de référence est réalisée par calcul algébrique et/ou par recherche dans une base de données.

Préférentiellement, l'organe de mesure et l'organe de calcul appartiennent à une même unité électronique, de préférence, une carte électronique. Cela permet de réduire la masse et l'encombrement.

Préférentiellement, l'organe de mesure et l'organe de comparaison appartiennent à une même unité électronique, de préférence, une carte électronique. Cela permet de réduire la masse et l'encombrement.

De manière préférée, le dispositif comprenant au moins trois électrodes de charge et de mesure définissant au moins une électrode de référence et au moins deux électrodes de test, l'organe de mesure est configuré pour mesurer la différence de potentiels électrolytiques entre l'électrode de référence et chaque électrode de test. L'utilisation de plusieurs électrodes de test permet d'obtenir plusieurs mesures afin de déterminer plusieurs indicateurs de santé de l'utilisateur ou obtenir de manière plus pertinente et fiable un indicateur de santé de l'utilisateur.

De préférence, l'électrode de référence est unique. Le nombre de mesures est avantageusement optimisé pour une masse et un encombrement réduits.

De manière préférée, au moins deux électrodes de test sont dans des matériaux différents de manière à mesurer au moins deux différences de potentiels électrolytiques différentes. De manière avantageuse, la différence de potentiels électrolytiques est fonction de la nature du matériau de l'électrode de test utilisée pour la mesure.

Préférentiellement, l'ensemble des électrodes de test sont dans des matériaux différents.

De manière préférée, le matériau d'au moins une électrode de charge et de mesure comprend au moins un métal de la liste suivante : Ti, Zr, Hf, V, Nb, Cr, Mo, W, Mn, Fe, Ru, Co, Rh, Ir, Ni, Pd, Cu, Ag, Au, Al, Sn, Zn et Pt ou un alliage comprenant un métal de la liste précédente ou un composé métallique issu d'un métal de la liste précédente. De manière avantageuse, le matériau d'une électrode de charge et de mesure conduit l'électricité pour remplir la fonction de charge et est adapté pour la mesure de différence de potentiels électrolytiques souhaitée.

Préférentiellement, le dispositif comprend au moins une électrode de mesure. L'électrode de mesure permet avantageusement de compléter les mesures réalisées par les électrodes de charge et de mesure, pour mesurer de manière plus fiable et précise l'état de santé de l'utilisateur.

Préférentiellement, le matériau d'au moins une électrode de mesure comprend un polymère, de la céramique ou un verre conducteur d'ions.

L'invention concerne également un bracelet configuré pour être porté au poignet de l'utilisateur et comprenant un boîtier monté fixe sur ledit bracelet, ledit boîtier comprenant une face intérieure configurée pour être en contact avec le poignet de l'utilisateur lorsque le bracelet est porté et une face extérieure opposée à la face intérieure, ledit bracelet comprenant le dispositif tel que décrit précédemment, monté dans le boîtier, les électrodes de charge et de mesure étant montées sur la face intérieure, de manière à être en contact avec la sueur (S) du poignet de l'utilisateur.

L'invention concerne également un vêtement configuré pour être porté par l'utilisateur et comprenant un envers configuré pour être en contact avec la peau de l'utilisateur lorsque le vêtement est porté, un endroit opposé à l'envers, ledit vêtement comprenant le dispositif tel que décrit précédemment, fixé sur l'envers de manière à être en contact avec la sueur de l'utilisateur.

Préférentiellement, le vêtement est un t-shirt.

Préférentiellement, le vêtement est une ceinture.

Préférentiellement, le vêtement est un sous-vêtement.

Préférentiellement, le vêtement est une chaussette.

Préférentiellement, le vêtement est une semelle.

L'invention concerne en outre un procédé de mesure de l'état de santé d'un utilisateur portant un dispositif tel que décrit précédemment, le procédé comprenant une étape de mesure par l'organe de mesure en coopération avec les électrodes de charge et de mesure d'au moins un paramètre physique de la sueur de l'utilisateur afin de mesurer son état de santé.

De manière préférée, le procédé comprend une étape de calcul, au cours de laquelle l'organe de calcul détermine au moins un indicateur de santé de l'utilisateur à partir du paramètre physique de la sueur.

De manière préférée, le procédé comprend une étape de comparaison, au cours de laquelle l'organe de comparaison compare l'indicateur de santé avec un seuil de référence.

Préférentiellement, le procédé comprend une étape de prévention de l'utilisateur, au cours de laquelle l'organe de comparaison émet une alarme sonore, visuelle ou autre, lorsque l'indicateur de santé I dépasse le seuil de référence I-ref de manière à informer l'utilisateur sur son état de santé.

De manière préférée, le procédé comprend une étape de charge, au cours de laquelle la batterie électrique 3 est reliée à un dispositif de charge afin d'être rechargée en électricité.

### PRESENTATION DES FIGURES

L'invention sera mieux comprise à la lecture de la description qui va suivre, donnée uniquement à titre d'exemple, et se référant aux dessins annexés donnés à titre d'exemples non limitatifs, dans lesquels des références identiques sont données à des objets semblables et sur lesquels :
La figure 1 est une représentation schématique d'un utilisateur portant un bracelet selon l'invention,
La figure 2 est une représentation schématique du bracelet selon l'invention,
La figure 3 est une représentation schématique du boîtier du bracelet comprenant le dispositif de mesure portable de l'état de santé de l'utilisateur selon l'invention et du fonctionnement en charge de la batterie électrique,
La figure 4 est une représentation schématique du boîtier du bracelet comprenant le dispositif de mesure portable de l'état de santé de l'utilisateur selon l'invention et du fonctionnement en décharge de la batterie électrique,
La figure 5 est une représentation schématique du boîtier du bracelet comprenant le dispositif de mesure portable de l'état de santé de l'utilisateur selon l'invention et du fonctionnement des électrodes de charge et de mesure en coopération avec l'organe de mesure,
La figure 6 est une représentation schématique du boîtier du bracelet comprenant le dispositif de mesure portable de l'état de santé de l'utilisateur selon l'invention et du fonctionnement de l'organe de calcul et de l'organe de comparaison,
La figure 7 est une représentation schématique d'un t-shirt comprenant le dispositif de mesure portable de l'état de santé de l'utilisateur selon l'invention,
La figure 8 est une représentation schématique d'une ceinture comprenant le dispositif de mesure portable de l'état de santé de l'utilisateur selon l'invention,
La figure 9A,
La figure 9B et
La figure 9C sont une représentation schématique d'un utilisateur portant la ceinture selon l'invention, à la taille, à la cheville et à l'arrière-bras respectivement et
La figure 10A,
La figure 10B et
La figure 10C sont des représentations schématiques de la disposition des électrodes de mesure du dispositif de mesure portable selon trois formes de réalisation de l'invention.

Il faut noter que les figures exposent l'invention de manière détaillée pour mettre en œuvre l'invention, lesdites figures pouvant bien entendu servir à mieux définir l'invention le cas échéant.

### DESCRIPTION DETAILLEE DE L'INVENTION

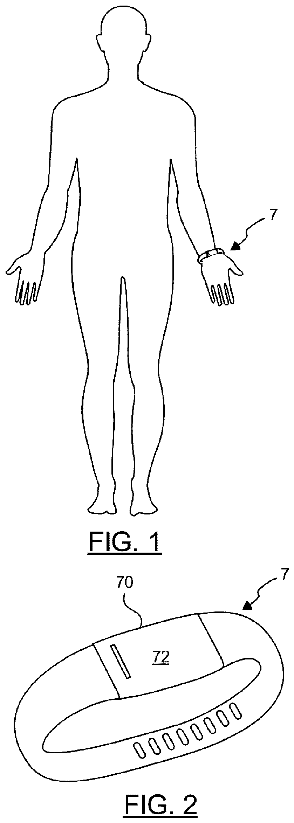

Il va être présenté un dispositif de mesure portable de l'état de santé selon l'invention. En référence aux figures 1 à 6, il est présenté une montre connectée comprenant un bracelet 7 comprenant un dispositif portable selon l'invention. Cette invention s'applique toutefois à tout vêtement ou analogue configuré pour être porté par l'utilisateur et en contact avec sa peau, tel qu'une chaussette, une ceinture, une semelle, un sous-vêtement, une bande adhésive, etc.

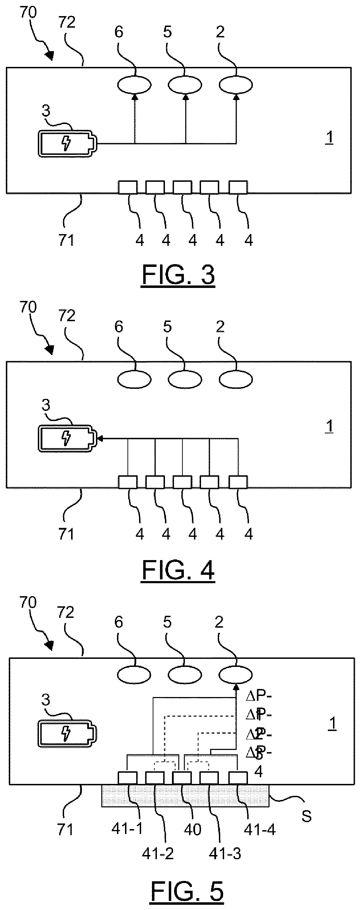

Dans l'exemple des figures 1 et 2, le bracelet 7 est configuré pour être porté au poignet de l'utilisateur et comprend un boîtier 70 monté sur le bracelet 7. Comme illustré sur la figure 3, le boîtier 70 comprend une face intérieure 71 configurée pour être en contact avec le poignet de l'utilisateur lorsque le bracelet 7 est porté, une face extérieure 72 opposée à la face intérieure et le dispositif 1 de mesure portable de l'état de santé d'un utilisateur monté dans le boîtier 70. Le bracelet 7 possède de manière connue une forme annulaire et comporte un système de fermeture, par exemple, à crans.

Selon l'invention, en référence aux figures 3 et 4 représentant le boîtier 70 de manière schématique en coupe longitudinale, le dispositif 1 comprend un organe de mesure 2 et une batterie électrique 3 alimentant électriquement l'organe de mesure 2. La batterie électrique 3 comprend dans cet exemple cinq électrodes de charge 4 configurées pour coopérer avec un dispositif de charge (non représenté) afin de recharger électriquement ladite batterie électrique 3. Selon l'invention, la batterie électrique 3 comprend au moins deux électrodes de charge 4. Le dispositif de charge peut être un chargeur électrique branché au réseau électrique via une prise à titre d'exemple. Autrement dit, la batterie électrique a deux modes de fonctionnement, à savoir un mode de fonctionnement en charge où elle est alimentée par le dispositif de charge (Figure 4) et un mode de fonctionnement en décharge où elle alimente l'organe de mesure 2 (Figure 3). Il va de soi que le dispositif 1 peut comprendre plusieurs batteries électriques 3, ce qui augmente cependant l'encombrement et la masse dudit dispositif 1. De même, le dispositif 1 peut comprendre plusieurs organes de mesure 2, de manière à réaliser des mesures de natures différentes.

Selon l'invention, en référence à la figure 5, au moins deux des électrodes de charge 4 sont positionnées sur la face intérieure 71 du boîtier 70 afin d'être en contact avec la sueur S du poignet de l'utilisateur. Dans cet exemple, les cinq électrodes 4 sont positionnées sur la face intérieure 71. Ces électrodes de charge 4, dénommées par la suite électrodes de charge et de mesure, mesurent en coopération avec l'organe de mesure 2 au moins un paramètre physique de la sueur S du poignet de l'utilisateur, suivant une deuxième fonction. Autrement dit, les électrodes de charge et de mesure ont deux fonctions : une première fonction de charge en coopération avec le dispositif de charge (Figure 4) et une seconde fonction de mesure en coopération avec l'organe de mesure 2 (Figure 5). Dans l'exemple de la figure 5, l'ensemble des électrodes de charge 4 sont des électrodes de charge et de mesure, afin de réaliser avantageusement un plus grand nombre de mesures. Il va toutefois de soi que certaines électrodes de charge 4 peuvent être dédiées uniquement à la fonction de charge. Par ailleurs, dans l'exemple de la figure 5, le dispositif 1 comprend cinq électrodes de charge 4 mais il va de soi que le nombre d'électrodes de charge 4 est quelconque. De préférence, ce nombre est suffisamment grand pour réaliser diverses mesures et suffisamment petit pour limiter l'encombrement et la masse.

Selon un aspect de l'invention, comme illustré sur la figure 5, l'organe de mesure 2 se présente sous la forme d'un voltmètre relié aux électrodes de charge et de mesure et est configuré pour mesurer une pluralité de différences de potentiels électrolytiques ΔP à partir de la sueur S de l'utilisateur. Dans l'exemple de la figure 5, l'organe de mesure 2 est unique afin de limiter la masse et l'encombrement du dispositif 1 mais il va de soi que le dispositif 1 peut comprendre plusieurs organes de mesure 2. Par ailleurs, il va de soi que l'organe de mesure 2 peut se présenter sous une forme autre qu'un voltmètre, tel qu'un ohmmètre ou un ampèremètre à titre d'exemples. Par suite, l'organe de mesure 2 peut être configuré pour mesurer un paramètre physique quelconque de la sueur S, autre qu'une différence de potentiels électrolytiques ΔP, tel que la résistance ou l'intensité électrique associée à cette différence de potentiels électrolytiques ΔP.

En pratique, un potentiel électrolytique correspond au potentiel émis par la réactivité chimique d'une ou plusieurs espèces chimiques contenues dans la sueur S et connues de l'homme du métier sous le terme d'ions, avec une ou plusieurs espèces chimiques d'une électrode de charge et de mesure. Une différence de potentiels électrolytiques ΔP correspond ainsi à une tension électrique égale à la différence entre un premier potentiel électrolytique associé à une première électrode de charge et de mesure et un deuxième potentiel électrolytique associé à une deuxième électrode de charge et de mesure. On décrit par la suite plus précisément une électrode de charge et de mesure, en particulier sa fonction de mesure.

Dans les faits, une électrode de charge et de mesure comprend un corps comprenant des molécules qui réagissent spontanément, suivant une réaction chimique connue de l'homme du métier sous le terme de réaction d'oxydoréduction, avec certains des ions de la sueur S. Le couple formé par un ion et une molécule du corps de l'électrode de charge et de mesure réagissant ensemble est connu de l'homme du métier sous le terme de couple oxydoréducteur. Les ions sont chargés électriquement si bien que la réaction d'oxydoréduction produit une tension électrique spontanée, correspondant à la différence de potentiels électrolytiques ΔP.

Selon un aspect de l'invention et en référence à la figure 5, l'organe de mesure 2 est ainsi configuré pour mesurer la différence de potentiel électrolytique d'un couple oxydoréducteur associé à une première électrode de charge et de mesure, dite électrode de test 41, par rapport à celui d'un autre couple oxydoréducteur, dit couple oxydoréducteur de référence et associé à une deuxième électrode de charge et de mesure, dite électrode de référence 40.

De préférence, tel que dans l'exemple de la figure 5, une des électrodes de charge et de mesure forme une électrode de référence 40 tandis que les autres électrodes de charge et de mesure forment des électrodes de test 41, afin de réaliser une mesure de différence de potentiels électrolytiques ΔP entre l'électrode de référence 40 et chaque électrode de test 41. L'encombrement et la masse du dispositif 1 sont ainsi avantageusement optimisés. Toutefois, il va de soi que plusieurs électrodes de charge et de mesure peuvent former des électrodes de référence 40. Les électrodes de charge et de mesure sont métalliques.

Selon un aspect de l'invention, le matériau des électrodes de charge et de mesure comprend au moins un métal de la liste suivante : Ti, Zr, Hf, V, Nb, Cr, Mo, W, Mn, Fe, Ru, Co, Rh, Ir, Ni, Pd, Cu, Ag, Au, Al et Sn ou un alliage comprenant un métal de la liste précédente ou un composé métallique issu d'un métal de la liste précédente. A titre d'exemple, l'électrode de charge et de mesure comporte une couche renouvelable de dioxyde de ruthénium RuO₂, du dioxyde de manganèse MnO₂ ou encore du phosphate de zinc Zn₃(PO₄)₂.

De préférence, le corps de chaque électrode de charge et de mesure est dans un matériau différent afin de mesurer les différences de potentiels électrolytiques ΔP de couples oxydoréducteurs différents de manière à suivre l'état de santé de l'utilisateur de façon globale et diversifiée. A titre d'exemple, en référence à la figure 5, l'électrode de référence 40 est en platine, une première électrode de test 41-1 est en fer, une deuxième électrode de test 41-2 est en magnésium, une troisième électrode de test 41-3 est en argent recouvert d'une couche de chlorure d'argent et une troisième électrode de test 41-4 est en zinc. Ces électrodes de test 41 sont ainsi configurées pour réagir chimiquement avec les ions ferreux, les ions magnésium, les ions chlorure et les ions zinc de la sueur S respectivement. Toutefois, il va de soi que le matériau de plusieurs électrodes de charge et de mesure peut être identique.

Selon un aspect de l'invention, le dispositif 1 comprend en outre une ou plusieurs électrodes de mesure, uniquement dédiées à la mesure de différences de potentiels électrolytiques ΔP. De préférence, le nombre d'électrodes de mesure est réduit pour limiter l'encombrement et la masse du dispositif 1. Selon un aspect, ces électrodes de mesure comprennent un corps dont le matériau est un polymère, de la céramique ou un verre conducteur d'ions. Les matériaux cités ne sont en effet pas métalliques et ne peuvent pas être utilisés pour réaliser une charge. De manière avantageuse, ces électrodes de mesure permettent de mesurer l'état de santé de l'utilisateur de manière complémentaire aux électrodes de charge et de mesure.

Selon un aspect de l'invention, les électrodes de mesure, pouvant également être dédiées à la charge, possèdent une grande surface de contact avec la peau de l'utilisateur pour que les mesures de différences de potentiels électrolytiques ΔP, c'est-à-dire des mesures potentiométriques, soient précises et facilement interprétables. Selon un autre aspect, pour des mesures ampérométriques, à savoir l'intensité associée à la différence de potentiels électrolytiques ΔP, les électrodes de mesure, pouvant également être dédiées à la charge, possèdent une faible surface de contact avec la peau de l'utilisateur pour que les densités de courant mesurées soient élevées et donc facilement interprétables. De préférence, les électrodes de mesure, pouvant également être dédiées à la charge, possèdent différentes surfaces de contact, afin de permettre à la fois des mesures potentiométriques et ampérométriques précises et facilement interprétables, ainsi qu'une plus grande diversité de mesures.

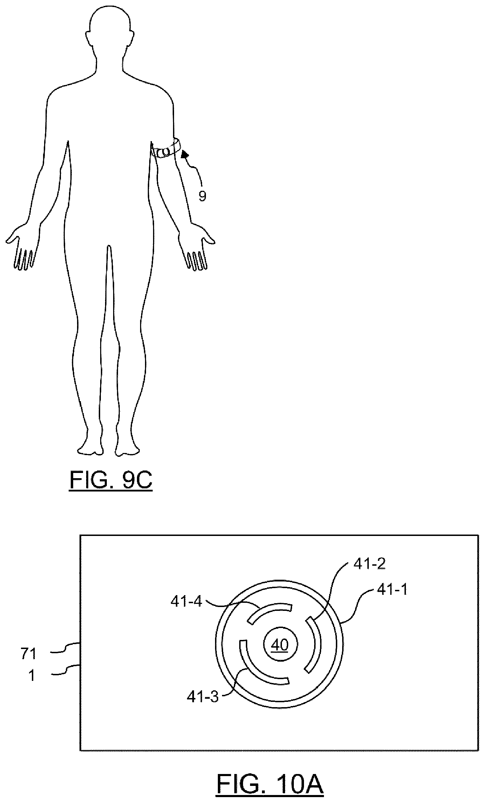

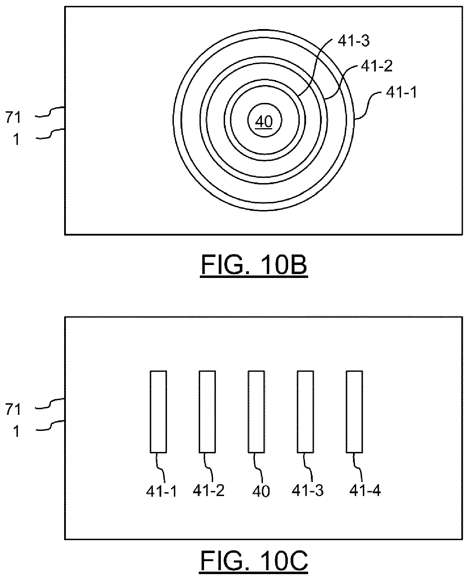

Préférentiellement, en référence aux figures 10A et 10B, les surfaces de contact des électrodes avec la peau de l'utilisateur se présentent sous la forme d'un disque entouré d'anneaux - ou de portions d'anneaux - concentriques, afin de limiter l'encombrement du dispositif 1 de mesure portable. Alternativement, dans l'exemple de la figure 10C, les surfaces de contact des électrodes avec la peau de l'utilisateur sont situées côte à côte pour limiter l'encombrement. De préférence, la distance séparant deux électrodes voisines est identique pour des mesures ampérométriques, notamment de sorte que les pertes ohmiques entre l'électrode de référence 40 et les différentes électrodes de test 41 soient identiques. Dans l'exemple de la figure 10A, en considérant que l'électrode de référence 41 possède la surface de contact en forme de disque centrale, les pertes ohmiques entre l'électrode de référence 40 et les électrodes de test 41 dont la surface de contact se présente sous la forme de portions d'anneau de même diamètre sont identiques. Par ailleurs, la fabrication et le montage d'électrodes de mesure de surfaces de contact concentriques est avantageusement simple.

De manière préférée, le dispositif 1 comprend en outre un capteur enzymatique afin d'améliorer la précision des mesures de différences de potentiels électrolytiques ΔP. Il va de soi que le dispositif 1 peut comprendre d'autres types de capteurs aidant à la précision desdites mesures.

De manière complémentaire, l'organe de mesure 2 peut être configuré pour mesurer une résistance de polarisation potentiostatique et/ou potentiodynamique - et/ou l'intensité et la différence de potentiel associée - au moyen d'au moins trois électrodes de mesure, pouvant également être dédiées à la charge. Plus précisément, dans le mode potentiostatique, l'organe de mesure 2 est configuré pour maintenir un potentiel fixe entre une électrode de référence et une électrode de test, la troisième électrode de mesure formant une électrode auxiliaire. Dans le mode potentiodynamique, l'organe de mesure 2 est au contraire configuré pour maintenir un potentiel variable de vitesse et de forme données. Le rôle des trois électrodes de mesure peut avantageusement être interverti pour diversifier les mesures. On considère par la suite un organe de mesure 2 uniquement configuré pour mesurer une différence de potentiels électrolytiques ΔP mais il va de soi que cette description est valable pour un organe de mesure 2 également configuré pour mesurer une résistance de polarisation potentiostatique et/ou potentiodynamique.

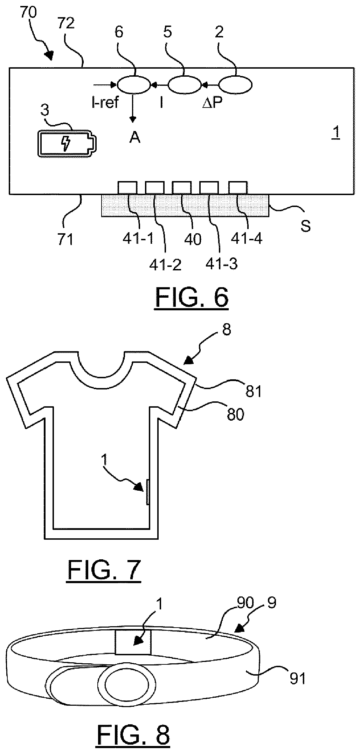

Selon un aspect de l'invention, comme illustré sur la figure 6, le dispositif 1 comprend également un organe de calcul 5 configuré pour déterminer, à partir d'une mesure de différence de potentiels électrolytiques ΔP, un indicateur de santé I de l'utilisateur. Cet indicateur de santé I de l'utilisateur peut à titre d'exemple être une concentration, une masse ou encore une quantité de matière d'un ion donné de la sueur S. En reprenant l'exemple de la figure 5, l'organe de calcul 5 détermine les concentrations en ions ferreux, en ions magnésium, en ions chlorure et en ions zinc de la sueur S à partir des mesures réalisées. De préférence, l'organe de calcul 5 est également configuré pour déterminer un indicateur de santé I de l'utilisateur à partir d'une pluralité de mesures de différences de potentiels électrolytiques ΔP. Ainsi, l'état de santé de l'utilisateur peut avantageusement être mesuré de façon plus diversifiée à partir d'un nombre réduit de mesures et donc d'électrodes de charge et de mesure. A titre d'exemple, la concentration en ions glutamate peut être déterminée de cette façon, avec l'aide du capteur enzymatique.

De manière préférée, l'organe de mesure 2 et l'organe de calcul 5 appartiennent à une même entité, telle qu'une carte électronique, afin de limiter la masse et l'encombrement du dispositif 1. Dans le cas où le bracelet est une montre connectée, l'organe de mesure 2 et l'organe de calcul 5 peuvent avantageusement former une même entité avec la carte électronique de ladite montre connectée dans cette même optique. De la même façon, la batterie électrique 3 du dispositif 1 peut avantageusement former une même entité avec la batterie électrique de ladite montre connectée.

Selon un autre aspect de l'invention, le dispositif 1 comprend également un organe de comparaison 6 configuré pour comparer un indicateur de santé I à un seuil de référence I-ref. De préférence, ledit organe de comparaison 6 est configuré pour prévenir l'utilisateur en cas de dépassement dudit seuil de référence I-ref. A titre d'exemple, si la concentration en ions ferreux est inférieure au seuil de référence, l'organe de comparaison 6 émet une alarme précisant que l'utilisateur peut souffrir d'une carence en fer à l'origine d'un état de fatigue. A titre d'exemple, si la concentration en ions magnésium est supérieure au seuil de référence, l'organe de comparaison 6 émet une alarme précisant que l'utilisateur peut souffrir de diarrhées et vomissements et lui recommande un avis médical. A titre d'exemple, si la concentration en ions glutamate est supérieure au seuil de référence, l'organe de comparaison 6 émet une alarme précisant que l'utilisateur peut souffrir de diabète. De manière avantageuse, l'utilisateur est prévenu rapidement et peut, si besoin, se faire soigner dans les meilleurs délais. L'organe de comparaison 6 peut émettre une alarme sonore, visuelle, lumineuse, informatique ou autre.

Préférentiellement, le seuil de référence I-ref est prédéterminé à l'aide de mesures de différences de potentiels électrolytiques préalables afin de réaliser une comparaison fiable, précise et propre à l'utilisateur. Il va toutefois de soi que le seuil de référence I-ref peut être obtenue de manière quelconque, par la théorie ou par des corrélations à titre d'exemple.

De manière préférée, l'organe de mesure 2 et l'organe de comparaison 6 forment une même entité, telle qu'une carte électronique, afin de limiter la masse et l'encombrement du dispositif 1. Dans le cas où le bracelet est une montre connectée, l'organe de mesure 2 et l'organe de comparaison 6 peuvent avantageusement former une même entité avec la carte électronique de ladite montre connectée dans cette même optique.

Selon un aspect de l'invention, le dispositif 1 comprend en outre un organe de programmation configuré pour commander de manière périodique la mesure de différence de potentiels électrolytiques ΔP par l'organe de mesure 2 en coopération avec les électrodes de charge et de mesure.

Selon un aspect de l'invention, le dispositif 1 comprend également un organe d'enregistrement configuré pour stocker en mémoire l'indicateur de santé I déterminés.

Selon un aspect de l'invention, le bracelet 7 peut être utilisé de manière déportée pour mesurer l'état de santé de l'utilisateur à partir d'un fluide corporel autre que la sueur S, tel que l'urine ou le sang. Autrement dit, le dispositif 1 permet la mesure de l'état de santé d'un utilisateur à partir d'un fluide corporel quelconque dudit utilisateur. Dans cette configuration, les électrodes de charge et de mesure doivent être placés au contact dudit fluide corporel pour réaliser la mesure. Cette configuration permet avantageusement à un utilisateur de pratiquer une mesure d'urgence et de pouvoir bénéficier d'un résultat rapide, sans avoir à se déplacer.

Il a été présenté une forme de réalisation de l'invention dans laquelle le dispositif 1 est monté sur un bracelet configuré pour être porté au poignet par l'utilisateur. Il va de soi que l'invention ne se limite pas à cette forme de réalisation mais englobe tout vêtement ou analogue configuré pour être porté par l'utilisateur et en contact avec sa peau.

Dans l'exemple de la figure 7, le dispositif 1 est ainsi monté sur un t-shirt 8, configuré pour être porté par l'utilisateur et comprenant un envers 80 configuré pour être en contact avec la peau de l'utilisateur lorsque le t-shirt 8 est porté, et un endroit 81 opposé à l'envers 80. Plus précisément, le dispositif 1 est configuré pour être fixé sur l'envers 81 du t-shirt. L'exemple de la figure 7 est adaptable à un vêtement quelconque en contact avec la peau de l'utilisateur, tel qu'un sous-vêtement, une chaussette, un pantalon, une semelle, etc.

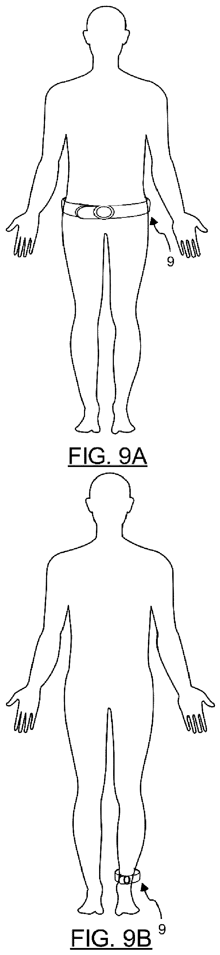

Dans l'exemple de la figure 8, le dispositif 1 est monté sur une ceinture 9, configurée pour être porté par l'utilisateur et comprenant un envers 90 configuré pour être en contact avec la peau de l'utilisateur lorsque la ceinture 9 est portée, et un endroit 91 opposé à l'envers 90. Plus précisément, le dispositif 1 est configuré pour être fixé sur l'envers 91 de la ceinture 9. Une telle ceinture 9 peut être portée à la taille, à la cheville ou à l'arrière bras à titre d'exemples non limitatifs représentés sur les figures 9A, 9B et 9C respectivement. De manière analogue, le dispositif 1 peut être monté sur une bande élastique ou une bande adhésive, configurée pour être placée directement sur la peau de l'utilisateur.

De manière avantageuse, le dispositif 1 selon l'invention peut être porté par l'utilisateur de diverses façons, suivant les préférences de chaque utilisateur. En outre, ce dispositif possède un encombrement, une masse et un coût réduits, ce qui peut séduire l'utilisateur.

L'invention concerne également un procédé de mesure de l'état de santé d'un utilisateur portant un dispositif 1 tel que décrit précédemment. Ce procédé comprend une étape de mesure au cours de laquelle l'organe de mesure 2 mesure en coopération avec les électrodes de charge et de mesure au moins un paramètre physique de la sueur S de l'utilisateur, afin de mesurer l'état de santé de l'utilisateur.

Selon un aspect de l'invention, l'organe de mesure 2 et les électrodes de charge et de mesure déterminent au moins une différence de potentiels électrolytiques ΔP.

De manière préférée, le procédé comprend une étape de calcul, au cours de laquelle l'organe de calcul 5 détermine au moins un indicateur de santé I de l'utilisateur à partir du paramètre physique de la sueur S.

De manière préférée, le procédé comprend une étape de comparaison, au cours de laquelle l'organe de comparaison 6 compare l'indicateur de santé I avec un seuil de référence I-ref. Préférentiellement, le procédé comprend une étape de prévention de l'utilisateur, au cours de laquelle l'organe de comparaison 6 émet une alarme lorsque l'indicateur de santé I dépasse le seuil de référence I-ref de manière à informer l'utilisateur sur son état de santé.

De manière préférée, le procédé comprend une étape de charge, au cours de laquelle la batterie électrique 3 est reliée à un dispositif de charge afin d'être rechargée en électricité.

Préférentiellement, l'étape de calcul suit l'étape de charge. De manière avantageuse, au cours de l'étape de charge, les électrodes de charge et de mesure sont en contact physique avec le dispositif de charge, ce qui permet de nettoyer la surface des électrodes de charge et de mesure, qui peuvent ainsi réaliser des mesures pertinentes.

On considère dans l'exemple qui suit un utilisateur possédant un bracelet 7 comprenant un dispositif 1 selon l'invention et qui souhaite suivre son état de santé pendant son jogging. Avant son jogging, comme illustré sur la figure 3, l'utilisateur branche la batterie électrique 3 du dispositif 1 à un chargeur électrique relié à une prise murale pour recharger la batterie électrique 3 via les électrodes de charge 4.

Une fois la batterie électrique 3 chargée, l'utilisateur attache le bracelet 7 à son poignet et appuie sur un bouton pour mettre en marche le dispositif 1, comme illustré sur la figure 4. Au moment de débuter son jogging, en référence aux figures 5 et 6, l'utilisateur appuie sur un bouton pour réaliser une première mesure de différences de potentiels électrolytiques ΔP de sa sueur S, permettant de déterminer sa concentration en acide lactique, son taux d'hydratation et son taux de sucre à titre d'exemples. Cette mesure définit son seuil de référence I-ref. Il programme ensuite le dispositif 1 de sorte qu'une mesure de différences de potentiels électrolytiques ΔP de sa sueur S soit réalisée toute les dix minutes durant son jogging. A miparcours, l'utilisateur reçoit une alerte sonore du dispositif 1 lui indiquant que son taux d'hydratation est faible et lui recommande une pause pour boire une certaine quantité d'eau. A la fin du parcours, l'utilisateur réalise une autre mesure et reçoit une alerte sonore lui indiquant que son taux de sucre est faible et que sa concentration en acide lactique grimpe, lui recommandant de manger une barre énergétique ainsi que d'effectuer une séance d'étirements pour éviter d'avoir des courbatures. Une fois le jogging terminé, l'utilisateur appuie sur un bouton pour éteindre le dispositif 1 et ôte le bracelet 7 de son poignet. Il peut également conserver un historique des mesures en vue de ses prochaines séances de jogging, permettant à l'organe de comparaison d'affiner le seuil de référence I-ref pour qu'il soit propre à l'utilisateur.

## Revendications

1. Dispositif (1) de mesure portable de l'état de santé d'un utilisateur configuré pour être porté par l'utilisateur et comprenant au moins un organe de mesure (2) et au moins une batterie électrique (3) alimentant électriquement l'organe de mesure (2), ladite batterie électrique (3) comprenant au moins trois électrodes de charge (4) configurées pour coopérer avec un dispositif de charge afin de recharger électriquement la batterie électrique (3), dispositif où au **moins** trois des électrodes de charge (4), dites électrodes de
charge et de mesure et définissant au moins une électrode de référence (40) et au moins deux électrodes de test (41), sont configurées pour être au contact de la sueur (S) de l'utilisateur lorsque le dispositif (1) est porté et sont reliées à l'organe de mesure (2) afin de mesurer, en coopération avec ledit organe de mesure (2), au moins un paramètre physique de la sueur (S) de l'utilisateur, à savoir la différence de potentiels électrolytiques (ΔP) entre l'électrode de référence (40) et chaque électrode de test (41), afin de mesurer l'état de santé de l'utilisateur.

2. Dispositif (1), selon la revendication 1, comprenant au moins un organe de calcul (5) configuré pour déterminer au moins un indicateur de santé (I) de l'utilisateur à partir de la différence de potentiels électrolytiques (ΔP).

3. Dispositif (1) selon la revendication 2, comprenant au moins un organe de comparaison (6) configuré pour comparer l'indicateur de santé (I) à au moins un seuil de référence (I-ref).

4. Dispositif (1), selon l'une des revendications 1 à 3, dans lequel au moins deux électrodes de test (41) sont dans des matériaux différents de manière à mesurer au moins deux différences de potentiels électrolytiques (ΔP) différentes.

5. Dispositif (1), selon l'une des revendications 1 à 4, dans lequel le matériau d'au moins une électrode de charge et de mesure comprend au moins un métal de la liste suivante : Ti, Zr, Hf, V, Nb, Cr, Mo, W, Mn, Fe, Ru, Co, Rh, Ir, Ni, Pd, Cu, Ag, Au, Al, Sn, Zn et Pt ou un alliage comprenant un métal de la liste précédente ou un composé métallique issu d'un métal de la liste précédente.

6. Bracelet (7), configuré pour être porté au poignet de l'utilisateur et comprenant un boîtier (70) monté fixe sur ledit bracelet (7), ledit boîtier (70) comprenant une face intérieure (71) configurée pour être en contact avec le poignet de l'utilisateur lorsque le bracelet (7) est porté et une face extérieure (72) opposée à la face intérieure (71), ledit bracelet (7) comprenant le dispositif (1), selon l'une des revendications 1 à 5, monté dans le boîtier (70), les électrodes de charge et de mesure étant montées sur la face intérieure (71), de manière à être en contact avec la sueur (S) du poignet de l'utilisateur.

7. Vêtement (8, 9), configuré pour être porté par l'utilisateur et comprenant un envers (80, 90) configuré pour être en contact avec la peau de l'utilisateur lorsque le vêtement (8, 9) est porté, un endroit (81, 91) opposé à l'envers (80, 90), ledit vêtement (8, 9) comprenant le dispositif (1), selon l'une des revendications 1 à 5, fixé sur l'envers (80, 90) de manière à être en contact avec la sueur (S) de l'utilisateur.

8. Procédé de mesure de l'état de santé d'un utilisateur portant un dispositif (1) selon l'une des revendications 1 à 5, le procédé comprenant une étape de mesure (E₁) par l'organe de mesure en coopération avec les électrodes de charge et de mesure d'au moins un paramètre physique de la sueur (S) de l'utilisateur afin de mesurer son état de santé.

## Patentansprüche

1. Tragbare Vorrichtung (1) zum Messen des Gesundheitszustands eines Benutzers, die ausgelegt ist, um von dem Benutzer getragen zu werden und mindestens ein Messorgan (2) und mindestens eine elektrische Batterie (3) umfasst, die das Messorgan (2) elektrisch versorgt, wobei die elektrische Batterie (3) mindestens drei Ladeelektroden (4) umfasst, die ausgelegt sind, um mit einer Ladevorrichtung zusammenzuwirken, um die elektrische Batterie (3) elektrisch aufzuladen, wobei bei der Vorrichtung mindestens drei der Ladeelektroden (4), bezeichnet als Lade- und Messelektroden und die mindestens eine Referenzelektrode (40) und mindestens zwei Testelektroden (41) definieren, ausgelegt sind, um mit dem Schweiß (S) des Benutzers im Kontakt zu sein, wenn die Vorrichtung (1) getragen wird und mit dem Messorgan (2) verbunden sind, um im Zusammenwirken mit dem Messorgan (2) mindestens einen physikalischen Parameter des Schweißes (S) des Benutzers zu messen, nämlich die Differenz elektrolytischer Potentiale (ΔP) zwischen der Referenzelektrode (40) und jeder Testelektrode (41), um den Gesundheitszustands des Benutzers zu messen.

2. Vorrichtung (1) nach Anspruch 1, die mindestens ein Rechenorgan (5) umfasst, das ausgelegt ist, um mindestens einen Gesundheitsindikator (I) des Benutzers ausgehend von der Differenz elektrolytischer Potentiale (ΔP) zu bestimmen.

3. Vorrichtung (1) nach Anspruch 2, die mindestens ein Vergleichsorgan (6) umfasst, das ausgelegt ist, um den Gesundheitsindikator (I) mit mindestens einem Referenzgrenzwert (I-ref) zu vergleichen.

4. Vorrichtung (1) nach einem der Ansprüche 1 bis 3, wobei mindestens zwei Testelektroden (41) derart aus unterschiedlichen Materialien sind, dass mindestens zwei unterschiedliche Differenzenelektrolytischer Potentiale (ΔP) gemessen werden.

5. Vorrichtung (1), nach einem der Ansprüche 1 bis 4, wobei das Material mindestens einer Lade- und Messelektrode mindestens ein Metall aus der folgenden Liste umfasst: Ti, Zr, Hf, V, Nb, Cr, Mo, W, Mn, Fe, Ru, Co, Rh, Ir, Ni, Pd, Cu, Ag, Au, AI, Sn, Zn und Pt oder eine Legierung, die ein Metall aus der vorangehenden Liste oder eine Metallzusammensetzung umfasst, die aus einem Metall der vorangehenden Liste hervorgegangen ist.

6. Armband (7), das ausgelegt ist, um am Handgelenk des Benutzers getragen zu werden und ein Gehäuse (70) umfasst, das an dem Armband (7) fest angebracht ist, wobei das Gehäuse (70) eine Innenfläche (71) umfasst, die ausgelegt ist, um mit dem Handgelenk des Benutzers im Kontakt zu sein, wenn das Armband (7) getragen wird, und eine Außenfläche (72) gegenüber der Innenfläche (71), wobei das Armband (7) die Vorrichtung (1) nach einem der Ansprüche 1 bis 5 umfasst, die im Gehäuse (70) angebracht ist, wobei die Lade- und Messelektroden auf der Innenfläche (71) derart angebracht sind, dass sie mit dem Schweiß (S) am Handgelenk des Benutzers im Kontakt sind.

7. Bekleidung (8, 9), die ausgelegt ist, um von dem Benutzer getragen zu werden und eine linke Seite (80, 90) umfasst, die ausgelegt ist, um mit der Haut des Benutzers im Kontakt zu sein, wenn die Bekleidung (8, 9) getragen wird, eine rechte Seite (81, 91) gegenüber der linken Seite (80, 90), wobei die Bekleidung (8, 9) die Vorrichtung (1), nach einem der Ansprüche 1 bis 5 umfasst, die auf der linken Seite (80, 90) derart befestigt ist, dass sie mit dem Schweiß (S) des Benutzers im Kontakt ist.

8. Verfahren zum Messen des Gesundheitszustands eines Benutzers, der eine Vorrichtung (1) nach einem der Ansprüche 1 bis 5 trägt, wobei das Verfahren einen Schritt des Messens (E₁) durch das Messorgan im Zusammenwirken mit den Lade- und Messelektroden mindestens eines physikalischen Parameters des Schweißes (S) des Benutzers umfasst, um seinen Gesundheitszustand zu messen.

## Claims

1. Device (1) for portable measurement of the state of health of a user configured to be worn by the user and comprising at least one measuring member (2) and at least one electrical battery (3) electrically supplying the measuring member (2), said electrical battery (3) comprising at least three charging electrodes (4) configured to cooperate with a charging device in order to electrically recharge the electrical battery (3), device wherein at least three of the charging electrodes (4), called charging and measuring electrodes and defining at least one reference electrode (40) and at least two test electrodes (41), are configured to be in contact with the user's sweat (S) when the device (1) is worn and are connected to the measuring member (2) in order to measure, in cooperation with said measuring member (2), at least one physical parameter of the user's sweat (S), namely the difference in electrolytic potentials (P) between the reference electrode (40) and each test electrode (41) in order to measure the state of health of the user.

2. Device (1), according to claim 1, comprising at least one calculation member (5) configured to determine at least one health indicator (I) of the user from the difference in electrolytic potentials (ΔP).

3. Device (1) according to claim 2, comprising at least one comparison member (6) configured to compare the health indicator (I) with at least one reference threshold (I-ref).

4. Device (1), according to one of claims 1 to 3, wherein at least two test electrodes (41) are made of different materials so as to measure at least two different electrolytic potential differences (ΔP).

5. Device (1), according to one of claims 1 to 4, wherein the material of at least one charging and measuring electrode comprises at least one metal from the following list: Ti, Zr, Hf, V, Nb, Cr, Mo, W, Mn, Fe, Ru, Co, Rh, Ir, Ni, Pd, Cu, Ag, Au, Al, Sn, Zn and Pt or an alloy comprising a metal from the preceding list or a metal compound derived from a metal from the preceding list.

6. Wrist strap (7), configured to be worn on the user's wrist and comprising a case (70) fixedly mounted on said wrist strap (7), said case (70) comprising an inner face (71) configured to be in contact with the user's wrist when the wrist strap (7) is worn and an outer face (72) opposite the inner face (71), said wrist strap (7) comprising the device (1), according to one of claims 1 to 5, mounted in the case (70), the charging and measuring electrodes being mounted on the inner face (71), so as to be in contact with the sweat (S) of the user's wrist.

7. Garment (8, 9), configured to be worn by the user and comprising a backside (80, 90) configured to be in contact with the user's skin when the garment (8, 9) is worn, a rigthside (81, 91) opposite to the backside (80, 90), said garment (8, 9) comprising the device (1), according to one of claims 1 to 5, attached on the backside (80, 90) so as to be in contact with the user's sweat (S).

8. Method for measuring the state of health of a user wearing a device (1) according to any one of claims 1 to 5, the method comprising a step of measuring (E₁) by the measuring member in cooperation with the charging and measuring electrodes of at least one physical parameter of the user's sweat (S) in order to measure his state of health.
